(19) Europäisches Patentamt
European Patent Office
Office européen des brevets

(11) Veröffentlichungsnummer: **0 048 911**
**B1**

(12) # EUROPÄISCHE PATENTSCHRIFT

(45) Veröffentlichungstag der Patentschrift: **05.12.84**

(51) Int. Cl.³: **C 07 C 131/00**, C 07 D 295/18, A 61 K 31/19

(21) Anmeldenummer: **81107441.8**

(22) Anmeldetag: **19.09.81**

(54) **O-substituierte alpha-Ketocarbonsäureoxime, Verfahren zu ihrer Herstellung, sowie Arzneimittel, die diese Verbindungen enthalten.**

(30) Priorität: **26.09.80 DE 3036281**

(43) Veröffentlichungstag der Anmeldung: **07.04.82 Patentblatt 82/14**

(45) Bekanntmachung des Hinweises auf die Patenterteilung: **05.12.84 Patentblatt 84/49**

(84) Benannte Vertragsstaaten: **AT BE CH DE FR GB IT LI LU NL SE**

(56) Entgegenhaltungen:
**DE - A - 2 837 863**
**US - A - 2 470 083**
**US - A - 3 104 258**
**US - A - 3 903 113**
**US - A - 4 052 194**

(73) Patentinhaber: **BOEHRINGER MANNHEIM GMBH, Patentabteilung, Abt. E Sandhofer Strasse 112-132 Postfach 31 01 20, D-6800 Mannheim 31 Waldhof (DE)**

(72) Erfinder: **Wolff, Hans, Dr.rer.nat., Rebenweg 24, D-6945 Hirschberg-Grossachsen (DE)**
Erfinder: **Heerdt, Ruth, Dr.rer.nat., Wallstädter Strasse 62, D-6800 Mannheim-Feudenheim (DE)**
Erfinder: **Hübner, Manfred, Dr.rer.nat., Saarlandstrasse 85e, D-6700 Ludwigshafen (DE)**
Erfinder: **Kühnle, Hans, Dr.ser.nat., Karlsruher-Strasse 17, D-6940 Weinheim (DE)**
Erfinder: **Schmidt, Felix, Prof.Dr.rer.nat., Stockacher Strasse 1, D-6800 Mannheim-Seckenheim (DE)**

**EP 0 048 911 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die vorliegende Erfindung betrifft O-substituierte α-Ketocarbonsäureoxime, deren physiologisch unbedenkliche Salze, Ester und Amide, Verfahren zu ihrer Herstellung, sowie Arzneimittel, die diese Verbindungen enthalten.

In der deutschen Offenlegungsschrift 27 26 210 sind Hydrazone der Brenztraubensäure mit hypoglykämischer Wirkung beschrieben, die Phenelzin (2-Phenylethylhydrazin) und ähnliche Verbindungen als Hydrazinkomponente enthalten. Mehrere der hierbei verwendeten Hydrazine sind als Monoaminooxidasehemmer bekannt. In hoher Dosierung kann durch sie eine Hypoglykämie hervorgerufen werden [Adnitt, P.I.: Hypoglycemic action of monoaminooxidase inhibitors, Diabetes 17: 628–633 (1980) u.a.]. Der genannten deutschen Offenlegungsschrift lag die Aufgabe zugrunde, den hypoglykämischen Effekt dieser Hydrazine zu verstärken und gleichzeitig die unerwünschte Monoaminooxidasehemmung aufzuheben. Die Lösung dieser Aufgabe wurde durch Kondensation der Hydrazine mit Brenztraubensäure erreicht.

Überraschenderweise wurde nun gefunden, dass α-Ketocarbonsäureoxime und deren Derivate, die als Hydroxylaminkomponente O-alkylierte Hydroxylamine enthalten, wertvolle pharmakologische Eigenschaften besitzen, die von den zugrundeliegenden Hydroxylaminen nicht bekannt sind. Gegenüber den literaturbekannten Brenztraubensäurehydrazonen zeichnen sich die erfindungsgemässen α-Ketocarbon-säureoxime durch ihre in physiologischen Medien hohe chemische Stabilität aus.

Die erfindungsgemässen Verbindungen hemmen insbesondere die intestinale Glucoseresorption. Ausserdem kann eine ausgeprägte hypoglykämische Wirkung beobachtet werden. Die Verbindungen eignen sich daher hervorragend zur Behandlung von Krankheiten, bei denen nach Aufnahme von kohlenhydrathaltigen Nahrungsmitteln starke und langanhaltende Hyperglykämien auftreten. Sie können somit als Therapeutika für die Indikationen Diabetes, Prädiabetes, Adipositas und Atherosklerose verwendet werden. Des weiteren zeigen sie eine lipidsenkende Wirkung.

Gegenstand der vorliegenden Erfindung sind daher Arzneimittel enthaltend α-Ketocarbonsäureoxime der allgemeinen Formel I

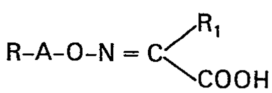 (I),

in welcher

R Wasserstoff, eine $C_3$–$C_8$-Cycloalkyl-, $C_1$–$C_6$-Alkoxy-, Cinnamyloxy-, Phenylamino-, Phenyl-N-alkylamino-, Phenylmercapto-, eine Aryl- oder Aryloxy-Gruppe, deren Aryl-Rest ein- oder mehrfach durch $C_{1-6}$ Alkyl, $C_{1-6}$ Alkoxy, Halogen, Hydroxy, Trifluormethyl, Amino, Acetylamino, Nitril, Nitro oder eine Methylendioxo-Gruppe substituiert sein kann,

A eine geradkettige oder verzweigte, gesättigte oder ungesättigte Alkylenkette mit bis zu 10 C-Atomen, die gegebenenfalls ein- oder mehrfach durch Halogen oder Hydroxy substituiert ist, wobei für den Fall, dass R einen Alkoxy- oder Aryloxyrest bedeutet, A ein geradkettiges Teilstück von mindestens 2 Kohlenstoffatomen besitzen muss, durch das der Rest R mit dem Sauerstoffatom verbunden ist, und

$R_1$ Wasserstoff, eine $C_1$–$C_8$ Alkylgruppe, die gegebenenfalls ein- oder mehrfach durch Halogen, Hydroxy, Nitril, Phenyl oder Carboxyl substituiert ist, Nitril oder Formyl bedeutet,

wobei R–A keine Methyl-, Ethyl- oder Benzylgruppe sein darf, oder deren physiologisch unbedenklichen Salze, Carbonsäureester und Amide, sowie an sich bekannte pharmakologisch verträgliche Trägerstoffe.

Für den Fachmann versteht es sich von selbst, dass für den Fall, dass R einen Alkoxy- oder Aryloxyrest bedeutet, A ein geradkettiges Teilstück von mindestens 2 Kohlenstoffatomen besitzen muss, durch das der Rest R mit dem Sauerstoffatom verbunden ist. Andernfalls wären in diesen Fällen unter der Formel I Acetale zu verstehen, die unter physiologischen Bedingungen instabil sind.

Im Zusammenhang mit der Synthese von α-Aminosäuren wurden die 2-Ethoxyiminopropionsäure (allgemeine Formel I; R = H; A = $-CH_2CH_2-$) und die 2-Benzyloxyiminopropionsäure (allgemeine Formel I; R = Phenyl; A = $-CH_2-$) als Zwischenprodukte hergestellt [W.E. Weaver et al., J. Org. Chem. 15, 741 (1950)]. Die 2-Methoxyiminopropionsäure (allgemeine Formel I; R = H; A = $-CH_2-$) ist bei der Derivatisierung der Brenztraubensäure zu analytischen Zwecken erhalten worden [Y. Ishitoya et al., Clin. Chim. Acta 27, 233 (1970)]. Eine pharmakodynamische Wirkung für diese Verbindungen ist bisher nicht beschrieben, so dass ihre Verwendung als Arzneimittel neu ist.

Weitere bekannte Verbindungen sind der Methylester der Cyano-methoxyimino-glyoxylsäure, der Ethylester der Cyano-ethoxyiminoglyoxylsäure (Beilstein 3 I, S. 775) und die 2-Benzyloxyimino-3-phenylpropionsäure (J. Org. Chem. 15, S. 741).

Aus der DE-A 28 37 863 sind ferner α-Ketocarbon-säureoximester und Amide zum Schutz von Pflanzenkulturen vor aggressiven Agrarchemikalien bekannt geworden. Eine pharmakologische Wirkung bei Mensch und Tier war jedoch noch nicht bekannt.

Gegenstand der vorliegenden Anmeldung sind demgegenüber die neuen α-Ketocarbon-säureoximderivate der allgemeinen Formel I′

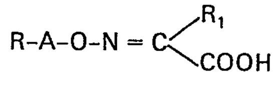 (I′),

in welcher

R Wasserstoff, eine $C_3$–$C_8$-Cycloalkyl-, Meth-

oxy-, Cinnamyloxy-, Phenylamino-, Phenyl-N-methylamino-, Phenylmercapto-, eine Aryl- oder Aryloxy-Gruppe, deren Aryl-Rest eine Phenyl- oder Naphthylgruppe ist, die ein- oder mehrfach durch $C_{1-6}$ Alkyl, $C_{1-6}$ Alkoxy, Halogen, Hydroxy, Trifluormethyl, Amino, Acetylamino, Nitril, Nitro oder eine Methylendioxo-Gruppe substituiert sein kann,

A eine geradkettige oder verzweigte gesättigte oder ungesättigte Alkylenkette mit 2 bis 8 C-Atomen, die gegebenenfalls durch eine Halogen- oder Hydroxygruppe substituiert ist, wobei für den Fall, dass R einen Alkoxy- oder Aryloxyrest bedeutet, A ein geradkettiges Teilstück von mindestens 2 Kohlenstoffatomen besitzen muss, durch das der Rest R mit dem Sauerstoffatom verbunden ist, und

$R_1$ Wasserstoff, eine $C_1$–$C_6$ Alkylgruppe, die gegebenenfalls ein- oder zweifach durch Halogen, Hydroxy oder Phenyl substituiert ist,

bedeutet, wobei R–A keine Alkylgruppe mit 2 bis 4 C-Atomen sein darf, sowie deren physiologisch unbedenklichen Salze, Carbonsäureester und Amide.

Unter Cycloalkylgruppen sind Carbocyclen mit 3 bis 8 Kohlenstoffatomen zu verstehen, vorzugsweise die Cyclohexylgruppe.

Unter Arylresten des Restes R werden aromatische Kohlenwasserstoffe mit 6 bis 14 Kohlenstoffatomen verstanden, bevorzugt die Phenyl- und die Naphthylgruppe.

Unter substituierten Arylresten werden solche aromatische Kohlenwasserstoffe mit 6 bis 14 Kohlenstoffatomen verstanden, die in einer oder mehreren Positionen die Hydroxygruppe, Halogen, die Trifluormethylgruppe, niedere Alkyl- oder niedere Alkoxygruppen, Amino-, Acetylamino-, Nitril-, Nitro- oder Methylendioxogruppen tragen. Insbesondere kommen dafür gegebenenfalls durch die vorgenannten Gruppen substituierte Phenyl- und Naphthylreste in Frage. Dabei versteht man unter Halogen Fluor, Chlor, Brom und Jod; vorzugsweise Fluor, Chlor und Brom.

Unter niederen Alkylgruppen sind in allen Fällen geradkettige oder verzweigte Reste mit 1 bis 6 Kohlenstoffatomen zu verstehen. Diese Definition gilt auch für den Alkylrest der niederen Alkoxygruppen. Vorzugsweise finden als niedere Alkylreste die Methyl- und die Butylgruppe und als niederer Alkoxyrest die Methoxygruppe Verwendung.

Der Aryloxyrest enthält aromatische Kohlenwasserstoffreste mit 6 bis 14 Kohlenstoffatomen, insbesondere den Phenylrest.

Unter unverzweigten Alkylenketten A seien in allen Fällen bevorzugt die folgenden verstanden:

$-(CH_2)_X-$mit X = 1-8, $-CH = CH-CH_2-$
und $-C \equiv C-CH_2-$.

Als verzweigte Gruppen A sind insbesondere bevorzugt die Gruppen:

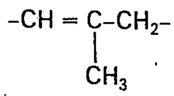
und
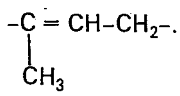
.

Als physiologisch unbedenkliche Salze kommen insbesondere Alkali-, Erdalkali- oder Ammoniumsalze (sowie gegebenenfalls Salze mit blutzuckersenkenden Biguaniden) in Frage, insbesondere das Natriumsalz.

Als Ester der Carbonsäuren der allgemeinen Formel I sind im Sinne der Erfindung generell die Reaktionsprodukte der Carbonsäuren mit Alkoholen zu verstehen. Bevorzugt sind als Alkoholkomponenten die niederen einwertigen Alkohole wie Methanol, Ethanol, Propanol oder Isopropanol.

Die erfindungsgemässen Amide der allgemeinen Formel I enthalten als Aminkomponenten z.B. Ammoniak, Mono- und Dialkylamine, z.B. 2-Hydroxyethylamin und 1-Methyl-piperazin sowie Aminosäuren, wobei p-Aminobenzoesäure, Anthranilsäure, Phenylalanin, α- und β-Alanin, Serin, Valin, Glycin, Arginin zu nennen sind.

Bevorzugte Verbindungstypen im Sinne der Erfindung sind 2-Cinnamyloxyimino-propionsäure-Derivate, wobei die Phenylkomponente auch substituiert sein kann. Ebenfalls bevorzugt sind Brenztraubensäure-Derivate, die in 3-Stellung der Propionsäure einen Fluor-Rest tragen.

Ferner sind Gegenstand der Erfindung sämtliche stereoisomeren Formen einer Verbindung der allgemeinen Formel I, die aufgrund der bei manchen Verbindungen vorhandenen asymmetrischen Kohlenstoffatome oder Doppelbindungen (C=C, C=N) auftreten können.

Gegenstand der vorliegenden Erfindungen sind weiterhin Verfahren zur Herstellung von α-Ketocarbonsäureoximen der allgemeinen Formel I', in der R, $R_1$ und A die oben angegebene Bedeutung haben, indem man zum Beispiel

a) ein Hydroxylamin der allgemeinen Formel II,

$R-A-O-NH_2$ (II)

in welcher R und A die oben angegebene Bedeutung haben, in an sich bekannter Weise mit einer Verbindung der allgemeinen Formel III,

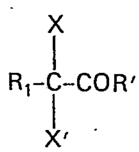
(III)

in welcher $R_1$ die angegebene Bedeutung hat, X und X' Halogen oder Alkoxygruppen oder zusammen ein Sauerstoffatom darstellen und R' Hydroxy, eine niedere Alkoxy- oder eine gegebenenfalls substituierte Aminogruppe bedeutet, umsetzt, oder

b) eine Verbindung der allgemeinen Formel IV

R-A-Y (IV)

in welcher R und A die oben angegebene Bedeutung haben und Y eine reaktive Gruppe darstellt, in an sich bekannter Weise mit einer Verbindung der allgemeinen Formel III, in welcher X und X'

zusammen die Gruppe =N-OH darstellen und $R_1$ und R′ die oben angegebene Bedeutung hat, umsetzt,
im Anschluss an die erfolgten Umsetzungen gegebenenfalls die erhaltenen Säurederivate in die freien Säuren der allgemeinen Formel I′ oder gewünschtenfalls die erhaltene freie Säure in einen Ester, in ein Amid oder in ein physiologisch verträgliches Salz umwandelt.

Halogen der Reste X und X′ der Formel III bedeutet Fluor, Chlor, Brom und Jod, vorzugsweise jedoch Chlor und Brom. Alkoxygruppen der Reste X, X′ und R′ sind Gruppen mit 1 bis 4 Kohlenstoffatomen, bevorzugt ist die Methoxy- und Ethoxygruppe. Als reaktive Verbindungen IV kommen inbesondere diejenigen in Frage, bei denen Y das Anion einer starken Säure, z.B. einer Halogenwasserstoff- oder Sulfonsäure darstellt. Bevorzugt werden Verbindungen der allgemeinen Formel IV verwendet, in denen Y eine p-Toluolsulfonyloxy-Gruppe, Chlor oder Brom bedeutet.

Nach dem unter a) genannten Verfahren wird das substituierte Hydroxylamin II oder ein entsprechendes Salz in einem geeigneten polaren Lösungsmittel (z.B. Wasser, einem niederen Alkohol oder Essigsäure) mit einer Verbindung der Formel III oder vorzugsweise mit dessen Salzen versetzt und gegebenenfalls mit Hilfe eines Puffers, z.B. Natriumacetat, auf einen schwach saueren pH-Wert gebracht. Die Reaktion läuft bei Raumtemperatur ab, kann jedoch zur Beschleunigung der Reaktionsgeschwindigkeit auch in der Wärme durchgeführt werden. Die Brenztraubensäureoxime I′ können als schwerlösliche Verbindungen aus dem Reaktionsmedium abfiltriert oder mit einem geeigneten unpolaren Lösungsmittel (wie z.B. Diethylether oder Methylenchlorid) extrahiert werden.

Nach dem unter b) genannten Verfahren wird eine reaktive Verbindung IV in einem geeigneten polaren Lösungsmittel (z.B. einem niederen Alkohol) mit einer Verbindung der Formel III, in welchem X und X′ zusammen die Gruppe =N-OH bedeuten, unter Zusatz eines basischen Kondensationsmittels (z.B. Alkalihydroxid oder Alkalialkoholat) vorzugsweise in der Wärme umgesetzt. Die Brenztraubensäureoxime können, gegebenenfalls nach Neutralisieren von überschüssigem Kondensationsmittel, durch geeignete Lösungsmittel direkt aus dem Reaktionsgemisch (z.B. durch unpolare Lösungsmittel wie Diethylether) oder aus dem Eindampfrückstand der Reaktionsmasse (z.B. durch polare Lösungsmittel wie niedere Alkohole) extrahiert werden.

Die Herstellung von Salzen der Carbonsäuren der allgemeinen Formel I′ erfolgt in an sich bekannter Weise, beispielsweise durch Umsetzung mit den entsprechenden freien Basen, Carbonaten oder Alkoholaten.

Die bei den oben aufgeführten Verfahren als Zwischenprodukte auftretenden Ester können isoliert oder gegebenenfalls direkt zu den entsprechenden Carbonsäuren verseift werden. Umgekehrt können die erhaltenen Carbonsäuren wieder nach an sich bekannten Methoden zu den gewünschten Estern umgesetzt werden. Die Verseifungen der Ester werden vorzugsweise in alkalischem Medium durchgeführt.

Die erfindungsgemässen Amide der allgemeinen Formel I′ können nach an sich bekannten Methoden aus den Carbonsäuren oder ihren reaktiven Derivaten durch Umsetzung mit Aminen hergestellt werden.

Die substituierten Hydroxylamine bzw. ihre Salze sind zum Teil neue Verbindungen. Eine Reindarstellung ist im allgemeinen nicht erforderlich, so dass die anfallenden Rohprodukte eingesetzt werden können. Sie können nach an sich bekannten Verfahren hergestellt werden, beispielsweise durch Umsetzung von Verbindungen der allgemeinen Formel IV mit N-Hydroxyphthalimid und nachfolgende Abspaltung der Phthalsäure-Schutzgruppe z.B. mittels Hydrazin oder mit Hydroxycarbamidsäureestern und anschliessende Verseifung der O-alkylierten Derivate.

Als erfindungsgemässe antidiabetische Zubereitungen kommen alle üblichen oralen und parenteralen Applikationsformen in Frage, beispielsweise Tabletten, Kapseln, Dragees, Sirupe, Lösungen, Suspensionen, Tropfen, Suppositorien etc. Zu diesem Zweck vermischt man den Wirkstoff mit festen oder flüssigen Trägerstoffen und bringt sie anschliesend in die gewünschte Form. Feste Trägerstoffe sind z.B.Stärke, Lactose, Methylcellulose, Talkum, hochdisperse Kieselsäure, höher-molekulare Fettsäuren (wie Stearinsäure), Gelatine, Agar-Agar, Calciumphosphat, Magnesiumstearat, tierische und pflanzliche Fette, feste hochmolekulare Polymere (wie Polyethylenglykole). Für die orale Applikation geeignete Zubereitungen können gewünschtenfalls Geschmacks- und Süssstoffe enthalten.

Als Injektionsmedium kommt vorzugsweise Wasser zur Anwendung, welches die bei Injektionslösungen üblichen Zusätze wie Stabilisierungsmittel, Lösungsvermittler und/oder Puffer enthält. Derartige Zusätze sind z.B. Acetat- oder Tartrat-Puffer, Ethanol, Komplexbildner (wie Ethylendiamin-tetraessigsäure und deren nichttoxische Salze) oder hochmolekulare Polymere (wie flüssiges Polyethylenoxid) zur Viskositätsregulierung.

Zur Bekämpfung von Krankheiten, bei denen nach Aufnahme von kohlenhydrathaltigen Nahrungsmitteln starke und langanhaltende Hyperglykämien auftreten, werden die pharmakologisch aktiven Verbindungen der allgemeinen Formel I in Einzeldosen von 10 bis 600, vorzugsweise von 50 bis 200 mg angewandt, wobei diese Einzeldosen je nach Bedarf ein- oder mehrmals pro Tag verabreicht werden können.

Bevorzugt im Sinne der vorliegenden Erfindung sind ausser den in den Beispielen genannten Verbindungen und den durch Kombination aller in den Ansprüchen genannten Bedeutungen der Substituenten ableitbaren Verbindungen noch insbesondere die folgenden Säuren bzw. ihre Salze sowie entsprechende Ester und Amide:

2-(3,5-Di-tert.-butyl-4-hydroxycinnamyloxyimino)propionsäure

2-(2-n-Propoxycinnamyloxyimino)propionsäure
2-(5-Chlor-3-methylcinnamyloxyimino)propionsäure

Die nachfolgenden Beispiele zeigen einige der zahlreichen Verfahrensvarianten, die zur Synthese der erfindungsgemässen Verbindungen verwendet werden können.

Beispiel 1

Natrium-2-(2-methallyloxyimino)propionat

In eine Lösung von 13,6 g (0,2 mol) Natriumethylat in 200 ml Ethanol werden unter Rühren zunächst 10,3 g (0,1 mol) Brenztraubensäureoxim eingetragen. Anschliessend tropft man in der Siedehitze langsam 18,1 g (0,2 mol) β-Methallylchlorid zu und hält noch 14 h auf dieser Temperatur. Nun werden erneut 9,0 g (0,1 mol) β-Methallylchlorid und 6,8 g (0,1 mol) Natriumethylat zugegeben und das Erhitzen weitere 7 h fortgesetzt. Danach saugt man heiss vom entstandenen Niederschlag ab und dampft das Filtrat ein. Den Eindampfrückstand kristallisiert man aus Ethanol um.
Ausbeute: 7,3 g (61% d.Th.); Fp. 240°C (Zers.).

In analoger Weise erhält man aus Brenztraubensäureoxim und

a) 2-Methoxyethylbromid
Natrium-2-(2-methoxyethyloxyimino)propionat
Fp. 185°C (Zers.)
b) 3-Phenylpropylbromid
Natrium-2-(3-phenylpropyloxyimino)propionat
Fp. 234°C (Zers.) (Ethanol-Wasser)
c) 2-Phenoxyethylbromid
Natrium-2-(2-phenoxyethyloxyimino)propionat
Fp. 209°C (Zers.) (Ethanol)
d) 2-Cyclohexylethylbromid
Natrium-2-(2-cyclohexylethyloxyimino)propionat
Fp. 234–235°C (Zers.) (Ethanol)

Beispiel 2

2-(2-p-Tolylethyloxyimino)propionsäure

Man setzt 2-p-Tolylethylbromid und Brenztraubensäureoxim nach dem in Beispiel 1 beschriebenen Verfahren um und dampft das Reaktionsgemisch nach Ablauf der Reaktion ein. Den Rückstand nimmt man in wenig Wasser auf, wäscht mit Ether und säuert die wässrige Lösung an. Der Niederschlag wird abgesaugt und getrocknet.
Ausbeute: 41% d.Th.; Fp. 106–108°C.

In analoger Weise erhält man aus Brenztraubensäureoxim und

a) 2-Phenylethylbromid
2-(2-Phenylethyloxyimino)propionsäure
Fp. 74°C
b) Hexylbromid
2-Hexyloxyiminopropionsäure
Fp. 41°C
c) Octylbromid
2-Octyloxyiminopropionsäure
Fp. 42°C (Ligroin)
d) 3-Phenylpropargylbromid
2-(3-Phenyl-2-propinyloxyimino)propionsäure
Fp. 84–85°C

Beispiel 3

2-(γ-Methylcinnamyloxyimino)propionsäure

Eine Suspension von 4,2 g (21 mmol) O-(γ-Methylcinnamyl)-hydroxylaminohydrochlorid in 50 ml Wasser wird mit 50 ml Methylenchlorid unterschichtet und anschliessend unter Rühren mit einer Lösung von 2,73 g (31 mmol) Brenztraubensäure und 2,54 g (31 mmol) Natriumacetat in 10 ml Wasser versetzt. Der Niederschlag löst sich dabei grösstenteils auf. Man rührt noch 1 h nach, trennt die Methylenchloridphase ab und extrahiert die wässrige Phase mit Methylenchlorid. Die vereinigten organischen Extrakte werden getrocknet und eingedampft. Den Rückstand kristallisiert man aus Ligroin um.
Ausbeute: 3,3 g (67% d.Th.); Fp. 56–58°C.

In analoger Weise erhält man aus Brenztraubensäure und

a) O-Cinnamylhydroxylaminhydrochlorid
2-Cinnamyloxyiminopropionsäure
Fp. 89–91°C
b) O-(β-Methylcinnamyl)hydroxylaminhydrochlorid
2-(β-Methylcinnamyloxyimino)propionsäure
Fp. 139°C

Beispiel 4

2-(3-Chlorcinnamyloxyimino)propionsäure

In eine Lösung von 7,8 g (42 mmol) O-(3-Chlorcinnamyl)hydroxylamin in 100 ml Methylenchlorid tropft man unter Rühren bei Zimmertemperatur eine Lösung von 5,6 g (64 mmol) Brenztraubensäure in 100 ml Wasser. Man rührt noch 15 min nach, trennt die organische Phase ab und extrahiert den wässrigen Teil noch einmal mit Methylenchlorid. Die vereinigten Methylenchloridlösungen werden getrocknet und eingedampft. Den Rückstand kristallisiert man aus einem Gemisch von Essigester und Ligroin um.
Ausbeute: 8,4 g (78% d.Th.); Fp. 99–101°C.

In analoger Weise erhält man aus Brenztraubensäure und

a) O-(2-Chlorcinnamyl)hydroxylamin
2-(2-Chlorcinnamyloxyimino)propionsäure
Fp. 88–90°C (Methanol-Wasser)
b) O-(4-Chlorcinnamyl)hydroxylamin
2-(4-Chlorcinnamyloxyimino)propionsäure
Fp. 114–116°C (Essigester-Ligroin)
c) O-Allylhydroxylamin
2-(Allyloxyimino)propionsäure
Fp. 38–39°C

Beispiel 5

2-(4-Fluorcinnamyloxyimino)propionsäure

Man setzt 8,2 g (49 mmol) O-(4-Fluorcinnamyl)hydroxylamin mit 5,2 g (59 mmol) Brenztraubensäure nach dem in Beispiel 4 beschriebenen Verfahren um.
Ausbeute: 6,2 g (53% d.Th.), Fp. 98–100°C (Essigester und Ligroin).

In analoger Weise erhält man aus Brenztraubensäure und

a) O-(3-Trifluormethylcinnamyl)hydroxylamin
2-(3-Trifluormethylcinnamyloxyimino)propionsäure
Fp. 103–105°C
b) O-(3-Methylcinnamyl)hydroxylamin
2-(3-Methylcinnamyloxyimino)propionsäure
Natriumsalz: Fp. 240°C (Zers.)
c) O-(4-tert.-Butylcinnamyl)hydroxylamin
2-(4-tert.-Butylcinnamyloxyimino)propionsäure
Fp. 114–116°C
d) O-(3-Methoxycinnamyl)hydroxylamin
2-(3-Methoxycinnamyloxyimino)propionsäure
Fp. 91–92°C
e) O-(2-Methoxycinnamyl)hydroxylamin
2-(2-Methoxycinnamyloxyimino)propionsäure
Fp. 84–86°C
f) O-(5-Chlor-2-methylcinnamyl)hydroxylamin
2-(5-Chlor-2-methylcinnamyloxyimino)propionsäure
Fp. 112°C
g) O-(5-Chlor-2-methoxycinnamyl)hydroxylamin
2-(5-Chlor-2-methoxycinnamyloxyimino)propionsäure
Fp. 124–126°C
h) O-[3-(1-Naphthyl)-2-propenyl]hydroxylamin
2-[3-(1-Naphthyl)-2-propenyloxyimino]propionsäure
Fp. 85–87°C
i) O-(3,5-Dichlorcinnamyl)hydroxylamin
2-(3,5-Dichlorcinnamyloxyimino)propionsäure
Fp. 101–104°C
j) O-(5-Chlor-2-methoxy-β-methylcinnamyl)hydroxylamin
2-(5-Chlor-2-methoxy-β-methylcinnamyloxyimino)propionsäure
Fp. 108–110°C
k) O-(5-Brom-2-methoxycinnamyl)hydroxylamin
2-(5-Brom-2-methoxycinnamyloxyimino)propionsäure
Fp. 118–120°C
l) O-(5-Fluor-2-methoxycinnamyl)hydroxylamin
2-(5-Fluor-2-methoxycinnamyloxyimino)propionsäure
Fp. 114–116°C
m) O-(2-Methoxy-5-trifluormethylcinnamyl)hydroxylamin
2-(2-Methoxy-5-trifluormethylcinnamyloxyimino)propionsäure
Fp. 120–123°C
n) O-(2,5-Dimethoxycinnamyl)hydroxylamin
2-(2,5-Dimethoxycinnamyloxyimino)propionsäure
Fp. 74–76°C
o) O-(2-Methoxy-5-methylcinnamyl)hydroxylamin
2-(2-Methoxy-5-methylcinnamyloxyimino)propionsäure
Fp. des Na-Salzes 218°C

Beispiel 6

2-(Cinnamyloxyimino)propionsäureethylester

Man erhitzt ein Gemisch von 2,2 g (10 mmol) 2-(Cinnamyloxyimino)propionsäure, 16 ml Chloroform, 1,78 g (15 mmol) Thionylchlorid und 2 Tropfen Dimethylformamid 2 Stunden auf Rückflusstemperatur. Dann dampft man das Chloroform und überschüssiges Thionylchlorid im Vakuum ab. Das zurückbleibende Öl versetzt man mit 20 ml Ethanol und lässt 12 Stunden bei Zimmertemperatur stehen. Danach dampft man das Ethanol im Vakuum ab und nimmt den Rückstand in Ether auf. Die Lösung wird zweimal mit gesättigter Natriumhydrogencarbonatlösung gewaschen, getrocknet und im Vakuum eingeengt. Den Rückstand löst man in Ligroin, klärt mit Kohle und engt wieder ein. Man erhält als Rückstand 2,4 g (97% d.Th.) 2-(Cinnamyloxyimino)propionsäureethylester, farbloses Öl, $n_D^{20} = 1,5390$.

Beispiel 7

2-(Cinnamyloxyimino)propionsäureamid

Man bereitet aus 4,38 g (20 mmol) 2-(Cinnamyloxyimino)propionsäure und 3,57 g (30 mmol) Thionylchlorid wie in Beispiel 6 beschrieben das 2-(Cinnamyloxyimino)propionylchlorid. Das rohe Säurechlorid wird in 20 ml Chloroform gelöst und unter Rühren zu einer eisgekühlten Lösung von Ammoniak in 40 ml Chloroform getropft. Das Reaktionsgemisch lässt man über Nacht bei Zimmertemperatur stehen und schüttelt dann mit 100 ml Wasser durch. Anschliessend wäscht man die organische Phase zunächst mit 0,5 N Salzsäure, dann mit gesättigter Natriumyhdrogencarbonatlösung, trocknet über Natriumsulfat und dampft im Vakuum ein. Den Rückstand kristallisiert man aus einem Gemisch von Essigester und Ligroin um. Man erhält 2,9 g (66% d.Th.) 2-(Cinnamyloxyimino)propionsäureamid, Fp. 120–121°C.

In analoger Weise erhält man aus 2-(Cinnamyloxyimino)propionylchlorid und Ethanolamin
a) 2-(Cinnamyloxyimino)propionsäure-N-(2-hydroxy-ethylamid)
Fp. 74–76°C

Beispiel 8

4-[2-(Cinnamyloxyimino)propionyl]-1-methylpiperazin

Man tropft zu einer Lösung von 2,0 g (20 mmol) 1-Methylpiperazin in 40 ml trockenem Pyridin bei 0°C unter Rühren 4,8 g (20 mmol) 2-(Cinnamyloxyimino)propionylchlorid (hergestellt gemäss Beispiel 6), rührt noch 1 Stunde bei 0°C nach und giesst auf Eis. Das Gemisch wird mit Ether extrahiert, die Etherextrakte werden über Natriumsulfat getrocknet und eingedampft.
Ausbeute: 4,3 g (71% d.Th.), farbloses Öl.

Das Hydrochlorid schmilzt bei 162–164°C (aus Isopropanol).

a) In analoger Weise erhält man aus β-Alaninethylester-hydrochlorid und 2-(Cinnamyloxyimino)propionylchlorid 3-[2-(Cinnamyloxyimino)-propionylamido]-propionsäureethylester, farbloses Öl.

13,7 g (43 mmol) des rohen Esters werden in 250 ml Methanol gelöst und mit 90 ml 1 N Kalilauge versetzt. Das Gemisch erhitzt man unter Rühren 2 Stunden auf 40°C und destilliert anschliessend im Vakuum das Methanol ab. Der wässrige Rückstand wird mit Kohle geklärt und mit 90 ml

1 N Salzsäure neutralisiert. Die ausgefallenen Kristalle werden abgesaugt und aus einem Gemisch von Essigester und Ligroin umkristallisiert. Man erhält 7,3 g (58% d.Th.) 3-[2-(Cinnamyloxyimino)propionylamido]propionsäure, Fp. 111–113°C.

Beispiel 9

2-(Cinnamyloxyimino)-3-fluorpropionsäure

Man tropft unter Rühren eine Lösung von 4,82 g (33 mmol) Natrium-3-fluor-2-oxopropionat-hydrat in 50 ml Wasser zu einer Lösung von 4,1 g (27,5 mmol) O-Cinnamylhydroxylamin in 50 ml Methylenchlorid. Dann versetzt man das Gemisch mit 33 ml (33 mmol) 1 N Salzsäure, rührt noch 30 Minuten nach und trennt die organische Phase ab. Man wäscht mit Wasser, trocknet über Natriumsulfat und dampft ein. Den Rückstand kristallisiert man zweimal aus Essigester und Ligroin um. Man erhält 3,1 g (48% d.Th.) 2-(Cinnamyloxyimino)-3-fluorpropionsäure, Fp. 81–83°C.

In analoger Weise erhält man aus

a) 3-Chlor-2-oxopropionsäure und O-Cinnamylhydroxylamin
3-Chlor-2-cinnamyloxyimino-propionsäure
Fp. 95–98°C

Beispiel 10

Cinnamyloxyiminoessigsäure

Man versetzt unter Rühren eine Lösung von 3,73 g (25 mmol) O-Cinnamylhydroxylamin in 50 ml Methylenchlorid mit einer Lösung von 2,22 g (30 mmol) Glyoxylsäure in 50 ml Wasser und rührt das Gemisch 30 Minuten bei Zimmertemperatur durch. Dann trennt man die Phasen ab und wäscht den organischen Teil mit Wasser, trocknet ihn mit Natriumsulfat und dampft ein. Den Rückstand kristallisiert man aus Essigester und Ligroin um. Man erhält 2,7 g (53% d.Th.) Cinnamyloxyiminoessigsäure, Fp. 85–88°C.

In analoger Weise erhält man aus O-Cinnamylhydroxylamin und

a) 2-Oxobuttersäure
2-Cinnamyloxyiminobuttersäure
Fp. 75–77°C
b) 3-Methyl-2-oxobuttersäure
2-Cinnamyloxyimino-3-methylbuttersäure
Natriumsalz: Fp. 220°C (Zers.)
c) 2-Oxovaleriansäure
2-Cinnamyloxyimino-valeriansäure
Fp. 53–55°C
d) 4-Methyl-2-oxovaleriansäure
2-Cinnamyloxyimino-4-methylvaleriansäure
Fp. 60–62°C
e) 3-Methyl-2-oxovaleriansäure
2-Cinnamyloxyimino-3-methylvaleriansäure
Fp. 57–59°C
f) 2-Oxo-octansäure
2-Cinnamyloxyimino-octansäure
Fp. 56–58°C
g) Phenylbrenztraubensäure
2-Cinnamyloxyimino-3-phenylpropionsäure
Fp. 84–86°C
h) Oxalessigsäure
2-Cinnamyloxyimino-bernsteinsäure
Fp. 124–126°C
i) α-Ketoglutarsäure
2-Cinnamyloxyimino-glutarsäure
Fp. 121–123°C.

Beispiel 11

Natrium-2-(4-phenylbutoxyimino)propionat

Eine Lösung von 5,0 g (30 mmol) O-(4-Phenylbutyl)hydroxylamin in 60 ml Methylenchlorid wird unter Rühren tropfenweise mit einer Lösung von 9,2 g (36 mmol) Brenztraubensäure in 60 ml Wasser versetzt. Man rührt noch 30 Minuten nach und trennt dann die Phasen. Die organische Phase wird über Natriumsulfat getrocknet und im Vakuum eingedampft. Als Rückstand verbleiben 7,1 g farbloses Öl, das sich beim Behandeln mit einer Lösung von 2,3 g (27,5 mmol) Natriumhydrogencarbonat in 50 ml Wasser unter Aufschäumen löst. Die wässrige Lösung wird mit Ether gewaschen, mit Kohle geklärt und im Vakuum eingedampft. Den Rückstand verreibt man unter Aceton. Man erhält 4,0 g (52% d.Th.) Natrium-2-(4-phenylbutoxyimino)propionat, Fp. 215–216°C.

In analoger Weise erhält man aus Brenztraubensäure und

a) O-(5-Phenylpentyl)hydroxylamin
Natrium-2-(5-phenylpentoxyimino)propionat
Fp. 209–212°C
b) O-(2-Phenoxypropyl)hydroxylamin
Natrium-2-(2-phenoxypropoxyimino)propionat
Fp. 204–206°C

Beispiel 12

2-(3-Phenoxypropoxyimino)propionsäure

Man setzt 5,0 g (30 mmol) O-(3-Phenoxypropyl)hydroxylamin mit 3,2 g (36 mmol) Brenztraubensäure nach dem in Beispiel 11 beschriebenen Verfahren um und kristallisiert den Eindampfrückstand aus Essigester und Ligroin um. Man erhält 6,1 g (86% d.Th.) 2-(3-Phenoxypropoxyimino)propionsäure, Fp. 86–88°C.

In analoger Weise erhält man aus Brenztraubensäure und

a) O-[2-(4-Chlorphenoxy)propyl]hydroxylamin
2-[2-(4-Chlorphenoxy)propoxyimino]propionsäure Fp. 70–73°C

Beispiel 13

2-Cinnamyloxyimino-propionsäure

Zu einer Lösung von 3,5 g (24 mmol) 2,2-Dichlorpropionsäure und 3,45 g (25 mmol) Kaliumcarbonat in 40 ml Wasser gibt man 4,0 g (28 mmol) O-(Cinnamyl)hydroxylamin, erhitzt unter Rühren auf 90°C, tropft innerhalb 30 Minuten eine Lösung von weiteren 3,45 g (25 mmol) Kaliumcarbonat in 15 ml Wasser zu und rührt 1 Stunde bei 90°C. Dann kühlt man ab, schüttelt mit Ether aus und säuert mit Salzsäure an. Der Niederschlag wird abgesaugt und aus einem Gemisch von Ligroin und Essigester umkristallisiert.
Ausbeute: 3,0 g (57% d.Th.), Fp. 89–91°C.
Der Mischschmelzpunkt mit dem in Beispiel 3a

beschriebenen Produkt zeigt keine Depression.

Beispiel 14

2-Cinnamyloxyiminopropionsäuremethylester

Zu 2,8 g (19 mmol) 2,2-Dimthoxypropionsäuremethylester in 100 ml Wasser werden 2,8 g (15 mmol) O-Cinnamylhydroxylaminhydrochlorid in 20 ml Wasser gegeben. Man lässt das Gemisch unter gelegentlichem Umschütteln 2 Tage stehen und extrahiert dann das entstandene Öl mit Methylenchlorid. Die Extrakte werden getrocknet und eingedampft. Man erhält als Rückstand 3,3 g (94% d.Th.) 2-Cinnamyloxyiminopropionsäuremethylester, farbloses Öl.

Der Ester kann mittels Kalilauge in Methanol zur 2-Cinnamyloxyiminopropionsäure, Fp. 89-91°C, verseift werden.

Beispiel 15

2-(3-Fluorcinnamyloxyimino)brenztraubensäure

Man setzt 6,2 g (37 mmol) O-(3-Fluorcinnamyl)hydroxylamin mit 3,9 g (44 mmol) Brenztraubensäure nach dem in Beispiel 4 beschriebenen Verfahren um.

Ausbeute: 5,4 g (61% d.Th.), Fp. 88-90°C (Essigester und Ligroin)

Durch geeignete Wahl der Ausgangskomponenten lassen sich in analoger Weise herstellen:

2-[3-(3-Methylphenyl)propoxyimino]propionsäure
2-[3-(4-Methylphenyl)propoxyimino]propionsäure
2-[3-(3-Chlorphenyl)propoxyimino]propionsäure
2-[3-(4-Chlorphenyl)propoxyimino]propionsäure
2-[3-(3-Methoxyphenyl)propoxyimino]propionsäure
2-[3-(4-Methoxyphenyl)propoxyimino]propionsäure
2-(2-Methyl-3-phenylpropoxyimino)propionsäure
2-[5-(3-Chlorphenyl)pentyloxyimino]propionsäure
2-[5-(4-Chlorphenyl)pentyloxyimino]propionsäure
2-[5-(4-Methoxyphenyl)pentyloxyimino]propionsäure
2-(3-Cyclohexyl-2-propenyloxyimino]propionsäure
2-(4-Methylcinnamyloxyimino)propionsäure
2-(3-t-Butylcinnamyloxyimino)propionsäure
2-(3-Bromcinnamyloxyimino)propionsäure, Fp. 107-109°C
2-(3-Cyanocinnamyloxyimino)propionsäure
2-(3,4-Methylendioxycinnamyloxyimino)propionsäure
2-(3-Nitrocinnamyloxyimino)propionsäure, Fp. 131-132°C
2-(3-Aminocinnamyloxyimino)propionsäure
2-(3-Acetamidocinnamyloxyimino)propionsäure
2-[3-(3-Methylphenyl)-2-propinyloxyimino]propionsäure
2-[3-(4-Methylphenyl)-2-propinyloxyimino]propionsäure
2-[3-(3-Chlorphenyl)-2-propinyloxyimino]propionsäure
2-[3-(4-Chlorphenyl)-2-propinyloxyimino]propionsäure
2-[3-(4-Methoxyphenyl)-2-propinyloxyimino]propionsäure
2-(3-Hydroxy-3-phenylpropoxyimino)propionsäure
2-(2-Hydroxy-3-phenylpropoxyimino)propionsäure
2-(2-Chlor-3-phenylpropoxyimino)propionsäure
2-(β-Chlorcinnamyloxyimino)propionsäure, Fp. 116-118°C
2-(3-Oxo-3-phenylpropoxyimino)propionsäure
2-(2-Oxo-3-phenylpropoxyimino)propionsäure
2-[2-(3-Chlorphenoxy)propoxyimino]propionsäure
2-[2-(4-Methoxyphenoxy)propoxyimino]propionsäure
2-[3-(3-Chlorphenoxy)propoxyimino]propionsäure
2-[3-(4-Chlorphenoxy)propoxyimino]propionsäure
2-[3-(4-Methoxyphenoxy)propoxyimino]propionsäure
2-(2-Hydroxy-3-phenoxypropoxyimino)propionsäure
2-(3-Anilino-propoxyimino)propionsäure
2-[3-(N-Methylanilino)propoxyimino]propionsäure
2-(3-Anilino-2-hydroxypropoxyimino)propionsäure
2-(3-Phenylthiopropoxyimino)propionsäure
2-(2-Cinnamyloxyethoxyimino)propionsäure
3-Fluor-2-(2-propenyloxyimino)propionsäure
2-(2-Propenyloxyimino)buttersäure
3-Fluor-2-hexyloxyiminopropionsäure
2-(2-Hexyloxyimino)buttersäure, Fp. 236-237°C (Na-Salz)
3-Hydroxy-2-(3-phenylpropoxyimino)propionsäure
3-Fluor-2-(3-phenylpropoxyimino)propionsäure
2-(3-Phenylpropoxyimino)buttersäure
2-[3-(3-Chlorphenyl)propoxyimino]-3-fluorpropionsäure
2-[3-(3-Chlorphenyl)propoxyimino]buttersäure
3-Fluor-2-[3-(3-methylphenyl)propoxyimino]propionsäure
2-[3-(3-Methylphenyl)propoxyimino]buttersäure
3-Oxo-2-cinnamyloxyimino-propionsäure
2-Cinnamyloxyimino-3,3-dichlor-propionsäure
2-Cinnamyloxyimino-3-cyano-propionsäure
2-Cinnamyloxyimino-4-cyano-buttersäure
2-Cinnamyloxyimino-3-hydroxy-propionsäure
2-(3-Chlorcinnamyloxyimino)-3-fluorpropionsäure, Fp. 92-94°C
2-(3-Chlorcinnamyloxyimino)buttersäure, Fp. 91-93°C
3-Fluor-2-(3-methylcinnamyloxyimino)propionsäure
2-(3-Methylcinnamyloxyimino)buttersäure
3-Hydroxy-2-(3-phenylpropin-2-yloxyimino)propionsäure
3-Fluor-2-(3-phenylpropin-2-yloxyimino)propionsäure
2-(3-Phenylpropin-2-yloxyimino)buttersäure
2-[3-(3-Chlorphenyl)propin-2-yloxyimino]-3-fluorpropionsäure
2-[3-(3-Chlorphenyl)propin-2-yloxyimino]buttersäure
3-Fluor-2-[3-(3-methylphenyl)propin-2-yloxyimino]propionsäure
2-[3-(3-Methylphenyl)propin-2-yloxyimino]buttersäure
3-Fluor-2-(2-phenoxypropoxyimino)propionsäure
2-(2-Phenoxypropoxyimino)buttersäure

**Patentansprüche**

1. α-Ketocarbonsäureoxime der Formel I′

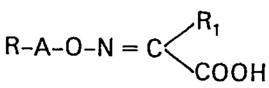

(I′),

in welcher

R Wasserstoff, eine $C_3$–$C_8$-Cycloalkyl-, Methoxy-, Cinnamyloxy-, Phenylamino-, Phenyl-N-methylamino-, Phenylmercapto-, eine Aryl- oder Aryloxy-Gruppe, deren Aryl-Rest eine Phenyl- oder Naphthylgruppe ist, die ein- oder mehrfach durch $C_{1-6}$ Alkyl, $C_{1-6}$ Alkoxy, Halogen, Hydroxy, Trifluormethyl, Amino, Acetylamino, Nitril, Nitro oder eine Methylendioxo-Gruppe substituiert sein kann,

A eine geradkettige oder verzweigte gesättigte oder ungesättigte Alkylenkette mit 2 bis 8 C-Atomen, die gegebenenfalls durch eine Halogen- oder Hydroxygruppe substituiert ist, wobei für den Fall, dass R einen Alkoxy- oder Aryloxyrest bedeutet, A ein geradkettiges Teilstück von mindestens 2 Kohlenstoffatomen besitzen muss, durch das der Rest R mit dem Sauerstoffatom verbunden ist, und

$R_1$ Wasserstoff, eine $C_1$–$C_6$ Alkylgruppe, die gegebenenfalls ein- oder zweifach durch Halogen, Hydroxy oder Phenyl substituiert ist,

bedeutet, wobei R–A keine Alkylgruppe mit 2 bis 4 C-Atomen sein darf, sowie deren physiologisch unbedenklichen Salze, Carbonsäureester und Amide.

2. Verbindungen gemäss Anspruch 1, dadurch gekennzeichnet, dass R und $R_1$ die angegebenen Bedeutungen haben und A eine $-CH_2CH_2-$, $-CH_2-CH_2-CH_2$, $-CH=CH-CH_2-$,

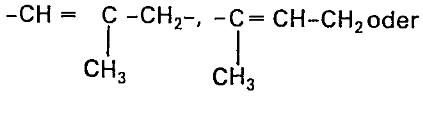

$-C\equiv C-CH_2-$ darstellt.

3. Verbindungen gemäss Anspruch 2, dadurch gekennzeichnet, dass R und A die angegebenen Bedeutungen haben und $R_1$ eine Methyl, Ethyl- oder Fluormethyl-Gruppe darstellt.

4. Verfahren zur Herstellung von α-Ketocarbon-säureoximen der allgemeinen Formel I′

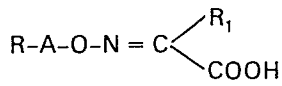

(I′),

in welcher

R Wasserstoff, eine $C_3$–$C_8$-Cycloalkyl-, Methoxy-, Cinnamyloxy-, Phenylamino-, Phenyl-N-methylamino-, Phenylmercapto-, eine Aryl- oder Aryloxy-Gruppe, deren Aryl-Rest eine Phenyl- oder Naphthylgruppe ist, die ein- oder mehrfach durch $C_{1-6}$ Alkyl, $C_{1-6}$ Alkoxy, Halogen, Hydroxy, Trifluormethyl, Amino, Acetylamino, Nitril, Nitro oder eine Methylendioxo-Gruppe substituiert sein kann,

A eine geradkettige oder verzweigte gesättigte oder ungesättigte Alkylenkette mit 2 bis 8 C-Atomen, die gegebenenfalls durch eine Halogen- oder Hydroxygruppe substituiert ist, wobei für den Fall, dass R einen Alkoxy- oder Aryloxyrest bedeutet, A ein geradkettiges Teilstück von mindestens 2 Kohlenstoffatomen besitzen muss, durch das der Rest R mit dem Sauerstoffatom verbunden ist, und

$R_1$ Wasserstoff, eine $C_1$–$C_6$ Alkylgruppe, die gegebenenfalls ein- oder zweifach durch Halogen, Hydroxy oder Phenyl substituiert ist,

bedeutet, wobei R–A keine Alkylgruppe mit 2 bis 4 C-Atomen sein darf, sowie deren physiologisch unbedenklichen Salze, Carbonsäureester und Amide, dadurch gekennzeichnet, dass man in an sich bekannter Weise

a) ein Hydroxylamin der allgemeinen Formel II,

$$R{-}A{-}O{-}NH_2 \qquad (II)$$

in welcher R und A die oben angegebene Bedeutung haben, mit einer Verbindung der allgemeinen Formel III,

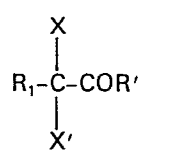

(III)

in welcher $R_1$ die angegebene Bedeutung hat, X und X′ Halogen oder Alkoxygruppen oder zusammen ein Sauerstoffatom darstellen und R′ Hydroxy, eine niedere Alkoxy- oder eine gegebenenfalls substituierte Aminogruppe bedeutet, umsetzt, oder

b) eine Verbindung der allgemeinen Formel IV

$$R{-}A{-}Y \qquad (IV)$$

in welcher R und A die oben angegebene Bedeutung haben und Y eine reaktive Gruppe darstellt, mit einer Verbindung der allgemeinen Formel III, in welcher X und X′ zusammen die Gruppe =N–OH darstellen und $R_1$ und R′ die oben angegebene Bedeutung hat, umsetzt, im Anschluss an die erfolgten Umsetzungen gegebenenfalls die erhaltenen Säurederivate in die freien Säuren der allgemeinen Formel I oder gewünschtenfalls die erhaltene freie Säure in einen Ester, in ein Amid

oder in ein physiologisch verträgliches Salz umwandelt.

5. Arzneimittel für Mensch und Tier, enthaltend α-Ketocarbonsäureoxime der Formel I

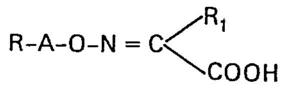

(I),

in welcher

R eine $C_3$–$C_8$-Cycloalkyl-, $C_1$–$C_6$-Alkoxy-, Cinnamyloxy-, Phenylamino-, Phenyl-N-alkylamino-, Phenylmercapto-, eine Aryl- oder Aryloxy-Gruppe, deren Aryl-Rest ein- oder mehrfach durch $C_{1-6}$ Alkyl, $C_{1-6}$ Alkoxy, Halogen, Hydroxy, Trifluormethyl, Amino, Acetylamino, Nitril, Nitro oder eine Methylendioxo-Gruppe substituiert sein kann,

A eine geradkettige oder verzweigte, gesättigte oder ungesättigte Alkylenkette mit bis zu 10 C-Atomen, die gegebenenfalls ein- oder mehrfach durch Halogen oder Hydroxy substituiert ist, wobei für den Fall, dass R einen Alkoxy- oder Aryloxyrest bedeutet, A ein geradkettiges Teilstück von mindestens 2 Kohlenstoffatomen besitzen muss, durch das der Rest R mit dem Sauerstoffatom verbunden ist, und

$R_1$ Wasserstoff, eine $C_1$–$C_8$ Alkylgruppe, die gegebenenfalls ein- oder mehrfach durch Halogen, Hydroxy, Nitril, Phenyl oder Carboxyl substituiert ist, Nitril oder Formyl bedeutet,

wobei R–A keine Benzylgruppe sein darf, oder deren physiologisch unbedenklichen Salze, Carbonsäureester, Salzen und Amide, sowie an sich bekannte pharmakologisch verträgliche Trägerstoffe.

6. Mittel, enthaltend α-Ketocarbonsäureoxime der Formel I

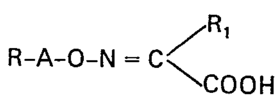

(I),

in welcher

R Wasserstoff

A eine geradkettige oder verzweigte, gesättigte oder ungesättigte Alkylenkette mit bis zu 10 C-Atomen, die gegebenenfalls ein- oder mehrfach durch Halogen oder Hydroxy substituiert ist, und

$R_1$ Wasserstoff, eine $C_1$–$C_8$ Alkylgruppe, die gegebenenfalls ein- oder mehrfach durch Halogen, Hydroxy, Nitril, Phenyl oder Carboxyl substituiert ist, Nitril oder Formyl bedeutet,

wobei R–A keine Methyl- oder Ethylgruppe sein darf, oder deren physiologisch unbedenklichen Salze, Carbonsäureester, Salzen, Estern und Amide, sowie an sich bekannte pharmakologisch verträgliche Trägerstoffe, zur Anwendung einer therapeutischen Behandlung des menschlichen oder tierischen Körpers.

7. α-Ketocarbonsäureoxime gemäss Anspruch 5 oder 6 zur Verwendung bei der Bekämpfung von Diabetes, Prädiabetes, Adipositas und Atherosklerose.

8. Verbindungen gemäss den Ansprüchen 1 bis 3 zur Verwendung bei der Bekämpfung von Diabetes, Prädiabetes, Adipositas oder Atherosklerose.

## Claims

1. α-Ketocarboxylic acid oximes of the formula I′

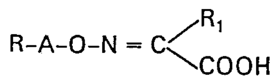

(I′),

in which R signifies hydrogen, a $C_3$–$C_8$ cycloalkyl, methoxy, cinnamyloxy, phenylamino, phenyl-N-methylamino, phenylmercapto, an aryl or aryloxy group, the aryl radical of which is a phenyl or naphthyl group which can be substituted one or more times by $C_{1-6}$ alkyl, $C_{1-6}$ alkoxy, halogen, hydroxyl, trifluoromethyl, amino, acetylamino, nitrile, nitro or a methylenedioxo group, A signifies a straight-chained or branched, saturated or unsaturated alkylene chain with 2 to 8 C-atoms, which is optionally substituted by a halogen or hydroxyl group, whereby for the case in which R signifies an alkoxy or aryloxy radical, A must possess a straight-chained part of at least 2 carbon atoms, through which the radical R is connected with the oxygen atom, and $R_1$ signifies hydrogen, a $C_1$–$C_6$ alkyl group, which is optionally substituted once or twice by halogen, hydroxyl or phenyl, whereby R–A cannot be an alkyl group with 2 to 4 C-atoms; as well as their physiologically acceptable salts, carboxylic acid esters and amides.

2. Compounds according to claim 1, characterised in that R and $R_1$ have the given meanings and A represents a $-CH_2-CH_2-$, $-CH_2-CH_2-CH_2-$, $-CH=CH-CH_2-$,

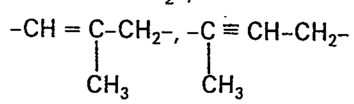

or $-C=C-CH_2-$.

3. Compounds according to claim 2, characterised in that R and A have the given meanings and $R_1$ represents a methyl, ethyl or fluoromethyl group.

4. Process for the preparation of α-ketocarboxylic acid oximes of the general formula I′

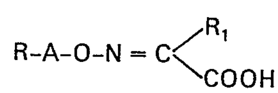

(I′),

in which R signifies hydrogen, a $C_3$–$C_8$ cycloalkyl, methoxy, cinnamyloxy, phenylamino, phenyl-N-methylamino, phenylmercapto, an aryl or aryloxy group, the aryl radical of which is a phenyl or naphthyl group which can be substituted one or more times by $C_{1-6}$ alkoxy, halogen, hydroxyl, trifluoromethyl, amino, acetylamino, nitrile, nitro or a methylenedioxo group, A signifies a straight-chained or branched, saturated or unsaturated alkylene chain with 2 to 8 C-atoms, which is optionally substituted by a halogen or hydroxyl group, whereby, for the case in which R signifies an alkoxy or aryloxy radical, A must possess a

straight-chained part of at least 2 carbon atoms through which the radical R is connected with the oxygen atom, and $R_1$ signifies hydrogen, a $C_1$–$C_6$ alkyl group, which is optionally substituted once or twice by halogen, hydroxyl or phenyl, whereby R–A cannot be an alkyl group with 2 to 4 C-atoms, as well as of their physiologically acceptable salts, carboxylic acid esters and amides, characterised in that, in per se known manner, one

a) reacts a hydroxylamine of the general formula II,

$R\text{-}A\text{-}O\text{-}NH_2$ (II),

in which R and A have the above-given meaning, with a compound of the general formula III

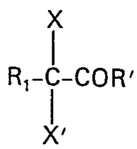

(III)

in which $R_1$ has the given meaning, X and X′ signify halogen or alkoxy groups or together represent an oxygen atom and R′ signifies hydroxyl, a lower alkoxy or an optionally substituted amino group; or

b) reacts a compound of general formula IV

R–A–Y (IV)

in which R and A have the above-given meaning and Y represents a reactive group, with a compound of general formula III, in which X and X′ together represent the group =N–OH and $R_1$ and R′ have the above-given meaning, subsequent to the reaction having taken place, one optionally converts the acid derivatives obtained into the free acids of the general formula I or, if desired, converts the free acid obtained into an ester, into an amide or into a physiologically acceptable salt.

5. Medicaments for humans and animals, containing α-ketocarboxylic acid oximes of the formula I

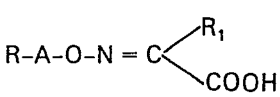

(I),

in which R signifies a $C_3$–$C_8$ cycloalkyl, $C_1$–$C_6$ alkoxy, cinnamyloxy, phenylamino, phenyl-N-alkylamino, phenylmercapto, an aryl or aryloxy group the aryl radical of which can be substituted one or more times by $C_{1-6}$ alkyl, $C_{1-6}$ alkoxy, halogen, hydroxyl, trifluoromethyl, amino, acetylamino, nitrile, nitro or a methylenedioxy group, A signifies a straight-chained or branched, saturated or unsaturated alkylene chain with up to 10 C-atoms, which is optionally substituted one or more times by halogen or hydroxyl, whereby, for the case in which R signifies an alkoxy or aryloxy radical, A must possess a straight-chained part through which the radical R is connected with the oxygen atom, and $R_1$ signifies hydrogen, a $C_1$–$C_8$ alkyl group, which is optionally substituted one or more times by halogen, hydroxyl, nitrile, phenyl or carboxyl, nitrile or formyl, whereby R–A cannot be a benzyl group, or their physiologically acceptable salts, carboxylic acid esters and amides, as well as per se known pharmacologically-acceptable carrier materials.

6. Agents containing α-ketocarboxylic acid oximes of the formula I

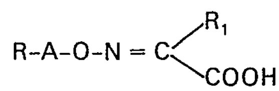

(I),

in which R signifies hydrogen, A signifies a straight-chained or branched, saturated or unsaturated alkylene chain with up to 10 C-atoms, which is optionally substituted one or more times by halogen or hydroxyl, and $R_1$ signifies hydrogen a $C_1$–$C_8$ alkyl group which is optionally substituted one or more times by halogen, hydroxyl, nitrile, phenyl or carboxyl, nitrile or formyl, whereby R–A cannot be a methyl or ethyl group; or their physiologically acceptable salts, carboxylic acid esters and amides, as well as per se known pharmacologically acceptable carrier materials for use in a therapeutic treatment of the human or animal body.

7. α-Ketocarboxylic acid oximes according to claim 5 or 6 for use in the combating of diabetes, prediabetes, adipositas and atherosclerosis.

8. Compounds according to claims 1 to 3 for use in the combating of diabetes, prediabetes, adipositas or atherosclerosis.

**Revendications**

1. Oximes d'acides carboxyliques α-cétoniques de formule I

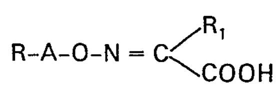

(I′),

dans laquelle:

R est de l'hydrogène, un groupe cyclo-alkyle $C_3$–$C_8$, méthoxy, cinnamyloxy, phénylamino, phényl-N-méthylamino, phénylmercapto, ou encore un groupe aryle ou aryloxy dont le reste aryle est un groupe phényle ou naphtyle pouvant être substitué une ou plusieurs fois par alkyle $C_{1-6}$, alcoxy $C_{1-6}$, halogène, hydroxyle, trifluorométhyle, amino, acétylamino, nitrile, nitro ou un groupe méthylène-dioxo,

A est une chaîne alkylène droite ou ramifiée, saturée ou insaturée ayant de 2 à 8 atomes de carbone, éventuellement substituée par un groupe halogène ou hydroxyle, A devant comporter, dans le cas où R est un reste alcoxy ou aryloxy, une fraction à chaîne droite ayant au moins 2 atomes de carbone, par laquelle le reste R est lié à l'atome d'oxygène, et

$R_1$ est de l'hydrogène, un groupe alkyle $C_1$–$C_6$ substitué éventuellement une ou deux fois

par de l'halogène, de l'hydroxyle ou du phényle,

R–A ne pouvant pas être un groupe alkyle ayant de 2 à 4 atomes de carbone, ainsi que leurs sels, esters carboxyliques et amides physiologiquement irréprochables.

2. Composés selon la revendication 1, caractérisés en ce que R et $R_1$ ont les significations indiquées et A est un groupe $-CH_2-CH_2-$, $-CH_2-CH_2-CH_2-$, $-CH=CH-CH_2-$,

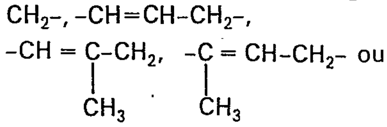 ou $-C\equiv C-CH_2-$.

3. Composés selon la revendication 2, caractérisés en ce que R et A ont les significations indiquées et $R_1$ est un groupe méthyle, éthyle ou fluorométhyle.

4. Procédé pour la préparation d'oximes d'acides carboxyliques α-cétoniques de formule I'

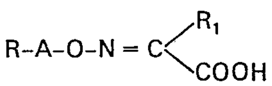 (I'),

dans laquelle:

R est de l'hydrogène, un groupe cyclo-alkyle $C_3$–$C_8$, méthoxy, cinnamyloxy, phénylamino, phényl-N-méthylamino, phénylmercapto, ou encore un groupe aryle ou aryloxy dont le reste aryle est un groupe phényle ou naphtyle pouvant être substitué une ou plusieurs fois par alkyle $C_{1-6}$, alcoxy $C_{1-6}$, halogène, hydroxyle, trifluorométhyle, amino, acétylamino, nitrile, nitro ou un groupe méthylène-dioxo,

A est une chaîne alkylène droite ou ramifiée, saturée ou insaturée ayant de 2 à 8 atomes de carbone, éventuellement substituée par un groupe halogène ou hydroxyle, A devant comporter, dans le cas où R est un reste alcoxy ou aryloxy, une fraction à chaîne droite ayant au moins 2 atomes de carbone, par laquelle le reste R est lié à l'atome d'oxygène, et

$R_1$ est de l'hydrogène, un groupe alkyle $C_1$–$C_6$ substitué éventuellement une ou deux fois par de l'halogène, de l'hydroxyle ou du phényle,

R–A ne pouvant pas être un groupe alkyle ayant de 2 à 4 atomes de carbone, ainsi que leurs sels, esters carboxyliques et amides physiologiquement irréprochables, caractérisé en ce que de façon en soi connue on fait réagir:

a) une hydroxylamine de formule générale II,

$R-A-O-NH_2$ (II)

dans laquelle R et A ont la signification indiquée ci-dessus, avec un composé de formule générale III

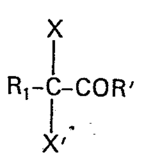 (III)

dans laquelle $R_1$ a la signification indiquée, X et X' sont de l'halogène ou des groupes alcoxy ou représentent ensemble un atome d'oxygène et R' est de l'hydroxyle, un groupe alcoxy inférieur ou un groupe amino éventuellement substitué, ou

b) un composé de formule générale IV

R–A–Y (IV)

dans laquelle R et A ont la signification indiquée ci-dessus et Y est un groupe réactif, avec un composé de formule générale III, dans laquelle X et X' représentent ensemble le groupe =N–OH et $R_1$ et R' ont la signification ci-dessus indiquée, et en ce qu'après l'achèvement de ces réactions on transforme éventuellement les dérivés d'acide obtenus en acides libres de formule générale I ou éventuellement les acides libres obtenus en un ester, en un amide ou en un sel physiologiquement acceptable.

5. Médicament pour l'homme et pour l'animal, contenant des oximes d'acides carboxyliques α-cétoniques de formule I

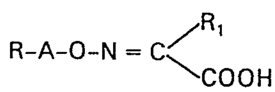 (I),

dans laquelle:

R est un groupe cyclo-alkyle $C_3$–$C_8$, alcoxy $C_1$–$C_6$, cinnamyloxy, phénylamino, phényl-N-alkylamino, phénylmercapto, un groupe aryle ou aryloxy dont le reste aryle peut être substitué une ou plusieurs fois par alkyle $C_{1-6}$, alcoxy $C_{1-6}$, halogène, hydroxyle, trifluorométhyle, amino, acétylamino, nitrile, nitro ou un groupe méthylène-dioxo,

A est une chaîne alkylène droite ou ramifiée, saturée ou insaturée ayant jusqu'à 10 atomes de carbone, substituée éventuellement une ou plusieurs fois par de l'halogène ou de l'hydroxyle, A devant comporter, dans le cas où R est un reste alcoxy ou aryloxy, une fraction à chaîne droite ayant au moins 2 atomes de carbone, par laquelle le reste R est lié à l'atome d'oxygène, et

$R_1$ est de l'hydrogène, un groupe alkyle $C_1$–$C_8$ éventuellement substitué une ou plusieurs fois par de l'halogène, de l'hydroxyle, du nitrile, du phényle ou du carboxyle, ou bien est un groupe nitrile ou formyle,

R–A ne pouvant pas être un groupe benzyle, ou leurs sels, esters carboxyliques et amides, physiologiquement irréprochables, ainsi que des matières de support connues pharmacologiquement acceptables.

6. Agent contenant des acides carboxyliques α-cétoniques de formule I

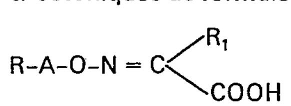 (I),

dans laquelle

R est de l'hydrogène

A est une chaîne alkylène droite ou ramifiée, saturée ou insaturée ayant jusqu'à 10 atomes de carbone, substituée éventuellement une ou plusieus fois par de l'halogène ou de l'hydroxyle, et

$R_1$ est de l'hydrogène, un groupe alkyle $C_1$–$C_8$, substitué éventuellement une ou plusieurs fois par de l'halogène, de l'hydroxyle, du nitrile, du phényle ou du carboxyle, ou bien est un groupe nitrile ou formyle,

R–A ne pouvant pas être un groupe méthyle ou éthyle, ou leurs sels, esters carboxyliques et amides physiologiquement irréprochables, ainsi que des matières de support connues pharmacologiquement acceptables, pour l'utilisation dans un traitement thérapeutique du corps de l'homme ou de l'animal.

7. Oximes d'acides carboxyliques α-cétoniques selon la revendication 5 ou 6 pour l'utilisation dans le traitement du diabète, du prédiabète, de l'adiposité et de l'athérosclérose.

8. Composés selon les revendications 1 à 3 pour l'utilisation dans le traitement du diabète, du prédiabète, de l'adiposité ou de l'athérosclérose.